# EUROPEAN PATENT SPECIFICATION

(11) **EP 4 369 655 B1**
(45) Date of publication and mention of the grant of the patent: **18.06.2025**
(21) Application number: 22205996.6
(22) Date of filing: 08.11.2022
(51) Int. Cl.: H04L 9/40, H04L 9/08, H04L 9/32, G16H 10/60, G16H 40/67

(54) **SYSTEM AND METHOD FOR SECURE HANDLING OF SENSITIVE DATA**
SYSTEM UND VERFAHREN ZUR SICHEREN HANDHABUNG VON SENSIBLEN DATEN
SYSTÈME ET PROCÉDÉ DE GESTION SÉCURISÉE DE DONNÉES SENSIBLES

(43) Date of publication of application: 15.05.2024
(73) Proprietor: IDUN Technologies AG, 8152 Opfikon (CH)
(72) Inventor: BURGER, Daniel, 8152 Glattpark (Opfikon) (CH); GISIN, Severine, 8152 Glattpark (Opfikon) (CH); BACHMANN, Simon, 8152 Glattpark (Opfikon) (CH); THIELEN, Moritz, 8152 Glattpark (Opfikon) (CH)
(74) Representative: Vossius & Partner Patentanwälte Rechtsanwälte mbB

(56) References cited:
- WO-A1-2018/111727
- WO-A1-2018/161051
- US-B1- 9 600 676

## Description

The human body is producing electrophysiological signals due to heart (ECG), muscle (EMG), eye (EOG) or brain (EEG) activity. These electric potentials can be measured using ExG recording devices, e.g. in-ear EEG devices using electrodes to measure the respective signals. These and any such health-related data are highly sensitive and high standards in view of data security and the safe handling of the measured data, i.e. the raw data, is of utmost importance to protect the user's privacy.

Current solutions of handling such sensitive data are not satisfying, because they are either too complex and not user friendly, do not provide sufficient functionalities in view of the handling of the data and/or the safe handling of the respective data is not sufficiently provided. For example, some of the currently available fitness trackers come with a proprietary app that needs to be installed to access the data. The device SDK (Software Development Kit) is the only method for getting real-time access to sensor data. Data ownership in these cases, i.e. the ownership of the raw data, is often unclear and/or not completely secure. There are no current solutions that provide a clear ownership of the data and a secure way of handling such sensitive data. WO 2018/161051 A1, WO 2018/111727 A1 and US 9 600 676 B1 are documents of the related art.

In view of the prior art, it is an object of the present disclosure to provide improved systems and methods for secure handling of sensitive data.

This object is achieved by the independent claims. Preferred variations and further developments are defined by the features of the dependent claims.

The present disclosure relates to a system for secure handling of sensitive data, the system comprises: a sensing device configured to measure electrophysiological signals and to encrypt the measured electrophysiological signals to generate encrypted electrophysiological signals using a public data key; a remote server configured to receive the encrypted electrophysiological signals from the sensing device and an encrypted private data key, wherein the remote server is further configured to decrypt the private data key based on a user's key phrase and to decrypt the encrypted electrophysiological signals based on the decrypted private data key to generate classified data based on the decrypted electrophysiological signals.

Various embodiments may preferably implement the following features:
Preferably, the public data key is provided on the sensing device and the private data key is provided on the external server.

The private data key is encrypted and wherein the user's key phrase is used to decrypt the private data key.

Preferably, the system further comprises: a user device configured to receive the encrypted electrophysiological signals from the sensing device and transmit the encrypted electrophysiological signals to the remote server. However, the sensing device may transmit the encrypted electrophysiological signals to the remote server directly without the user device being involved.

Preferably, the remote server is further configured to display the classified data on a user interface.

Preferably, the remote server is further configured to transmit the classified data to a user device.

Preferably, the user device comprises a display and wherein the user device is configured to display the classified data on the display.

Preferably, the remote server is further configured to receive instructions on how to generate the classified data, wherein the instructions preferably comprise information on one or more classifications from a set of classifications that are to be generated as the classified data.

Preferably, generating the classified data comprises transformation and filtering of the decrypted data.

The instructions are preferably provided by a user input on a web interface provided by the external server, wherein the web interface is preferably accessible for the user by way of the user key phrase (password).

Preferably, the web interface is accessible for the user by way of the user key phrase and a device identifier (ID).

Preferably, the device ID is a random value that is preferably automatically filled in by the software development kit (SDK) on the user device, so that the user does not have to fill in a (long) random value (such as yrj4dj5jtu3uc), but can concentrate only on the key phrase (password).

It is further preferred that the instructions are generated based on options selected by the user, so-called opt-in (options), which specify one or more classifications from a set of classifications that are to be generated as the classified data.

Preferably, the classified data are derived from the encrypted data received from the sensing device.

Preferably, the remote server is further configured to generate a token based on the user's key phrase and/or the classified data, wherein the token is preferably indicative of a successful reception of the user's key phrase and/or indicative on how to generate the classified data.

Preferably, the token comprises a predetermined or user specified validation time, wherein upon expiration of the validation time the remote server is configured to stop generating classified data.

Preferably, before the sensing device is used to measure electrophysiological signals, the sensing device comprises an initial public data key and the remote server comprises an initial private data key, wherein the initial private and public data keys are encrypted by a default random-generated device password, and wherein the remote server is configured to: generate a new encrypted private data key and a new public data key upon change, by a user, of the default random-generated device password to the user's key phrase; send the new public data key to the sensing device as the public data key; and store the new private data key on the external server as the private data key.

Preferably, after generation of the private key and the public key, the private key and the public key are provided with a predetermined lifetime, which upon expiry of said predetermined lifetime cause the private key and the public key to be inoperable.

Preferably, the predetermined lifetime is a timer that may specify a predetermined time span for which the private key and the public key are valid and which is preferably reset with every access of the user to the user device and/or the server such that the private key and the public key expire when the user does not access the user device and/or the server within the predetermined time span.

Preferably, the above-mentioned steps of generating a new private key and a new public key are repeated if the predetermined lifetime (timer) expired to create new private and public keys, which preferably again carry said lifetime. That is, after expiry of the predetermined lifetime (timer), the remote server is configured to: generate a new encrypted private data key and a new public data key upon access of the server by the user; send the new public data key to the sensing device as the public data key; and store the new private data key on the external server as the private data
The use of such a lifetime of the private and public key may further increase the security of the system.

Preferably, the user's key phrase is specific for a particular sensing device. This way, the external server can provide sensing device specific classifications based on the user's input without knowing the user behind it. Thus, the provider of the server has no knowledge about the user but only about the device.

The present disclosure also relates to a method for secure handling of sensitive data, the method including the following steps: measuring electrophysiological signals using a sensing device; encrypting the measured electrophysiological signals by means of a public data key provided on the sensing device; transmitting, by the sensing device, the encrypted electrophysiological signals to a remote server; receiving, by the remote server, the encrypted electrophysiological signals; decrypting the encrypted electrophysiological signals at the remote server based on a user's key phrase and a private data key; generating classified data based on the decrypted electrophysiological signals at the remote server.

Various embodiments may preferably implement the following features:
Preferably, the public data key is provided on the sensing device and the private data key is provided on the external server.

The private data key is encrypted and wherein the user's key phrase is used to decrypt the private data key.

Preferably, transmitting, by the sensing device, the encrypted electrophysiological signals to a remote server comprises transmitting the encrypted electrophysiological signals via a user device.

Preferably, the user device runs a third-party application (not from the provider of the sensing device and/or the server), wherein only a software development kit (SDK) from the provider of the sensing device and/or the server is added to the third-party application to allow communication between the user device (the application) and the sensing device/server.

Preferably, the method further comprises: displaying, by the remote server, the classified data on a user interface.

Preferably, the method further comprises: transmitting, by the remote server, the classified data to a user device.

Preferably, the method further comprises: displaying, by the user device, the classified data.

Preferably, generating classified data based on the encrypted electrophysiological signals at the remote server comprises: receiving, by the remote server, instructions on how to generate the classified data, wherein the instructions preferably comprise information on one or more classifications from a set of classifications that are to be generated as the classified data.

The instructions are preferably provided by a user input on a web interface provided by the external server, wherein the web interface is preferably accessible for the user by way of the user key phrase (password).

It is further preferred that the instructions are generated based on options selected by the user, so-called opt-in (options), which specify different classifications one or more classifications from a set of classifications that are to be generated as the classified data.

Preferably, the method further comprises: generating a token based on the user's key phrase and/or the classified data, wherein the token is preferably indicative of a successful reception of the user's key phrase and/or indicative on how to generate the classified data.

Preferably, the token comprises a predetermined or user specified validation time, wherein the method further comprises: stopping, by the remote server, to generating classified data upon expiration of the validation time.

Preferably, before the sensing device is used to measure electrophysiological signals, the method comprises: generating an initial private data key for the remote server and an initial public data key for the sensing device; encrypting the initial private and public data keys by a default random-generated device password; generating a new encrypted private data key and a new public data key upon change, by a user, of the default random-generated device password to the user's key phrase; storing the new public data key on the sensing device as the public data key; and storing the new private data key on the external server as the private data key.

Preferably, the method further comprises: receiving, by the external server, a validation request from the user device; validating, at the external server, the user device in case a predetermined condition is fulfilled.

Preferably, validating the user device comprises generating a token (API token) and sending the token to the user device.

Preferably, communication between the external server and the user device and/or the sensing device is allowed only for user devices having received the respective token.

Preferably, the presence of the respective token is checked by the external server and/or the sensing device before every communication with the user device.

Preferably, validating the user device is based on an access control list that specifies user devices that are allowed to communicate with the external server.

Preferably, the predetermined condition is whether the user device is comprised in the access control list.

Preferably, further comprising prohibiting communication between the external server and the user device and/or the sensing device when the predetermined condition is not fulfilled and/or in case no token is received by the user device.

The above-mentioned use of a predetermined lifetime is also applicable to the token described with regard to the validation procedure.

Preferably, wherein the user's key phrase is specific for a particular sensing device.

The term "classified" as used herein is understood in the sense of "classification" or "categorization" and not in the sense of "secret" or "confidential".

According to the above-described methods and systems a minimum of two parties are needed to decrypt the data (e.g. minimum 2 always including the user). For example, in case the sensing device and the external server are provided by a first party, the user may be the second party and the application provider is the third-party, the present disclosure requires the user's key phrase (input from the second party) and the private data key (stored on the first parties external server) in order to decrypt the data. Neither of the two parties has exclusive rights to decrypt the data. The provided classification options (opt-in's) and the data access is independent of the user and data ownership is with the sensing device and/or external server provider.

The above-described systems and methods provide an unobtrusive solution that can be integrated into a range of applications to extend their functionality with a sensing device with enabling the least privilege over the data: no raw data access to third-parties, no control of the measured raw data and full ownership of the raw data by the user based on opt-in functionalities. The least privilege in this context means: users have full control over their data, third-parties can access classified data from the measured raw data, but not the raw data itself, and the supplier of the measuring device and/or provider of the external server has access to the data but no control over it if there's no permission from the user, i.e. no key phrase and opt-in.

In fact, the present disclosure provides the following advantages. The opt-in of the various classifications cannot be bypassed by the third-party since this is handled by the user on a provided web application from the external server. A third-party has no access to the raw data since they have no access to the private data key and the user's key phrase. Even if a third-party would get access to the key phrase, they still cannot decrypt the raw data, because the private data key is missing. The service provider, i.e. the owner of the external server and/or provider of the sensing device, has no access to the raw data if the user has not given an opt-in and key phrase in order to decrypt the data on the external server. Users have no access to the raw data since they need to have access to the private data key, which is only available on the external server and never gets sent. If users do not register for an API key (API token), they cannot even send data to the external server. That is, the API key is generated by the provider of the external server and provide to the provider of the third-party application upon request. Therefore, only third-party applications that received a respective API key have the permission to exchange data with the external server. Thus, providing the provider of the external server with an additional option to control the access of the external server, e.g. to allow only certified third-party applications the right to exchange data with the external server. There is no need to download an additional application or driver to make this work, all of this can be done by including a lightweight SDK into the third-party application.

The exemplary embodiments disclosed herein are directed to providing features that will become readily apparent by reference to the following description when taken in conjunction with the accompany drawings. In accordance with various embodiments, exemplary systems, methods, devices and computer program products are disclosed herein. It is understood, however, that these embodiments are presented by way of example and not limitation, and it will be apparent to those of ordinary skill in the art who read the present disclosure that various modifications to the disclosed embodiments can be made while remaining within the scope of the present disclosure.

Thus, the present disclosure is not limited to the exemplary embodiments and applications described and illustrated herein. Additionally, the specific order and/or hierarchy of steps in the methods disclosed herein are merely exemplary approaches. Based upon design preferences, the specific order or hierarchy of steps of the disclosed methods or processes can be re-arranged while remaining within the scope of the present disclosure. Thus, those of ordinary skill in the art will understand that the methods and techniques disclosed herein present various steps or acts in a sample order, and the present disclosure is not limited to the specific order or hierarchy presented unless expressly stated otherwise.

Preferred embodiments of the present invention are further elucidated below with reference to the figures. The described embodiments do not limit the present invention.
- Fig. 1: shows, in a schematic view, a system for secure handling of sensitive data according to an embodiment of the present disclosure;
- Fig. 2: shows, in a schematic view, a system for secure handling of sensitive data according to an embodiment of the present disclosure;
- Fig. 3: shows a flow chart of a method for secure handling of sensitive data according to an embodiment of the present disclosure.

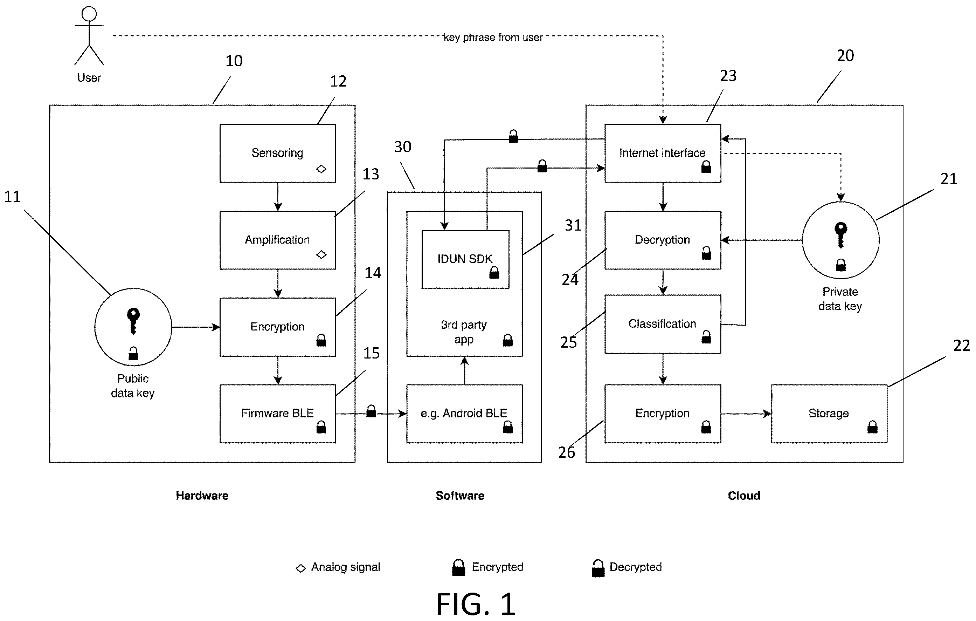

With reference to Fig. 1 a system for secure handling of sensitive data according to an embodiment of the present disclosure is described. Fig. 1 shows the sensing device 10, an external server 20 and a user device 30, wherein the presence of analog signals is indicated by a diamond shaped symbol, encrypted data is indicated by closed lock symbol and encrypted data is indicated by an open lock symbol.

The sensing device 10 is provided with a public data key 11 and sensoring equipment 12, e.g. electrode sensors. The sensoring equipment 12 is configured to sense electrophysiological signals, e.g. due to heart (ECG), muscle (EMG), eye (EOG) or brain (EEG) activity, or other sensitive data.

The signals measured by the sensoring equipment 12 may be amplified by an amplification unit 13 for further processing. Other signal processing steps are possible and the present disclosure is not limited to any specific signal processing, which will largely depend on the specific signals that are being processed. The skilled person will apply the necessary signal processing for the specific application based on his/her common general knowledge. A more detailed description thereof is therefore not necessary at this point.

That is, the amplifier receives data from the sensoring equipment 12, amplifies it and converts it into encrypted data. Minimal data processing may take place at this point as well, but the raw data is not changed, only put into a certain format (and encrypted). Furthermore, the data is buffered at this point (e.g. in case of a Bluetooth malfunction).

Next, the amplified signals are encrypted using the public data key 11 stored on the sensing device 10 such that the raw data is encrypted before being sent by the sensing device 10. The encryption may be done by an encryption unit 14. The use of public and private data keys and the respective encryption and decryption algorithms are generally known to the skilled person and the details thereof are therefore not further outlined at this point.

In order to send the encrypted data, the sensing device 10 may use any wireless or wired technology available. In Fig. 1, the encrypted data is sent to a user device 30, which receives the encrypted data. In this case, it is preferable that a wireless near field technology such as Bluetooth is used for sending the encrypted data from the sensing device 10 to the user device 30. In this case, the sensing device 10 may be provided with a firmware BLE (Bluetooth low energy) unit 15 and the user device 30 may be provided with a Bluetooth unit, e.g. an Android BLE unit. However, the user device may also be connected via a wired connection to the sensing device. The connection may also be a combination of a wired and wireless connection, e.g. via a base station of the user device and/or the sensing device.

The user device 30 may be any stationary or portable device in proximity to the sensing device 10. Examples for such a user device 30 are personal computers, laptops, tablets, mobile phones and the like. In an embodiment, the user device 30 is a mobile device, preferably a mobile phone.

The user device 30 runs a third-party application, which is configured to communicate with the external server 20 and configured to display classified data received from the external server 20 on the user device 30. The third-party application may incorporate a software development kit of the provider of the sensing device 10 and the external server 20 to provide the necessary communication capabilities.

Third-party in the context of this disclosure refers to a party or an entity different to the provider of the sensing device 10 and/or the external server 20. The third-party application running on the user device 30 may be supplemented only by necessary software for communication between the user device 30 and the sensing device 10 and/or the external server 20.

The user device 30 forwards the encrypted data received from the sensing device 10 to the external server 20. It should be appreciated that the encrypted data can also be send directly from the sensing device 10 to the external server 20. However, in this case an onboard memory (on the sensing device 10) may be needed to be available for buffering in case the sensing device 10 has no access to the internet. Therefore, it is preferred to connect the sensing device 10 to a user device 30, where it also works without an internet connection and where it can buffer as much data as is available on the user device 30 (which is usually multiple GBs).

The connection between the user device 30 and the external server 20 may be any wireless or wired technology available. Since the external server 20 is at a remote location, the connection may preferably be a wireless connection such as LTE, LTE-Advanced, 4G, 4G+ or 5G. However, in other cases the connection may be a broadband connection to connect the user device 30 to the external server 20, e.g. a personal computer may connect to the external server via a DSL (digital subscriber line) or a glass fiber connection. Combinations of wired and wireless connections are also possible, e.g. using a wireless router.

The encrypted data is received at the external server 20 via an interface, e.g. an internet interface 23. The encrypted data can be decrypted at the external server 20 using a private data key 21 provided on the external server 20. However, before the decryption can take place, a user key phrase is needed to decrypt the private data key 21, which is provided as an encrypted private data key 21 on the external server 20. That is, a user needs to provide a user key phrase to decrypt the private data key and thereby to permit the decryption of the encrypted raw data at the external server 20. The encryption may be executed by an encryption unit 24.

The private and public key pairs may be generated before the sensing device 10 gets sent to the user and are encrypted by a default random-generated key phrase which may then be changed as soon as the user sets up the device. After setting a new key phrase the user will create a new encrypted private data key for the external server 20 and will receive a new public data key on the sensing device 10.

The user also uses the key phrase to specify which classifications, from a set of predetermined classifications, are to be prepared and sent to the user device 30. That is, the external server 20 is configured to provide an independent web application, i.e. independent from the third-party application on the user device 30. The web application is provided for the user to register his/her sensing device 10 with an associated key phrase (password). The web application is further provided to enable the selection of different classifications. Thus, the user has full control over the decryption of the raw data and how the raw data is processed (classified) at the remote server 20 and the third-party application cannot influence these parameters.

If permitted by the user, by way of the user key phrase, the decrypted data is forwarded to the classification unit 25, which is configured to process the decrypted data and to generate classified data. The classified data is then sent to the user device 30, where the classified data may be displayed to the user.

The external server 20 may also comprise a storage 22 configured to store the classified data. Preferably, the external server encrypts the classified data (by way of an encryption unit 26) before storing the classified data in storage 22 and therefore stores only encrypted classified data in storage 22. The encryption is preferably also done with use of the public data key. For this encryption, the external server may have access to the public data key, e.g. using the sensing device for the encryption or temporarily accessing the public data key and subsequently deleting it.

In order to control access and/or communication between the user device 30 and the external server 20 and/or the sensing device 10, tokens may be used (e.g. JSON Web Tokens based approach). JSON Web Tokens are an open, industry standard RFC 7519 method for representing claims securely between two parties.

The token is generated at the external server 20 and send to the user device 30, where the token is saved. Only user devices 30 that are in possession of a respective token can access and/or communicate with the external server 20 and/or the sensing device 10. The token may also carry a timer, wherein upon expiry may prohibit access and/or communication between the user device 30 and the external server 20 and/or the sensing device 10 until a new token is generated. The timer may, for example, expire if the user has not logged into the external server 20 and/or the user device 30 before expiration of said timer.

Whereas if the user logs into the external server 20 and/or the user device 30 before expiration of said timer, said time may be reset.

All of the aforementioned units and their designated functions may be implemented using a processor that is configured to perform the respective functions.

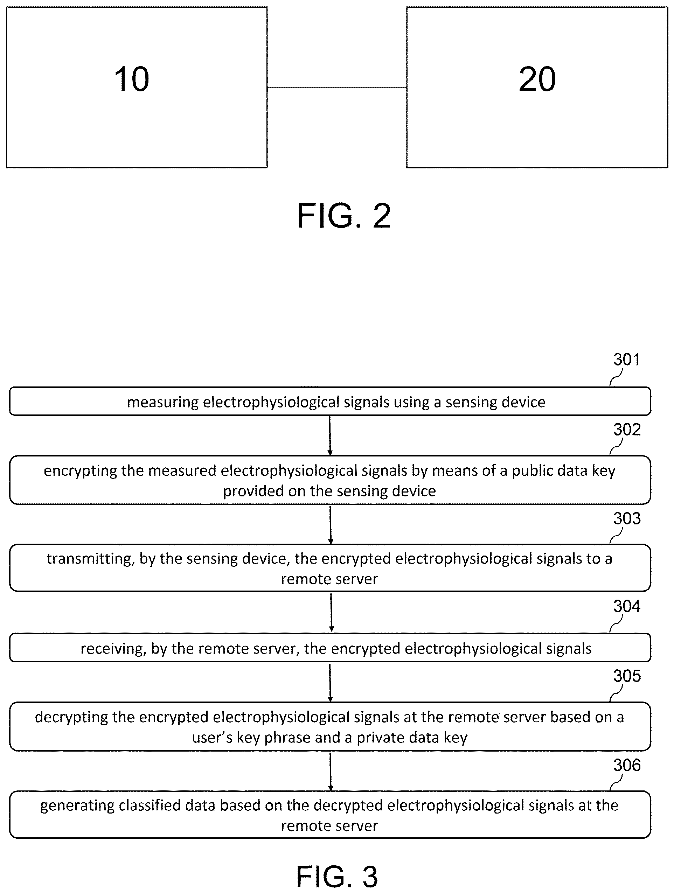

Fig. 2 shows a system according to an embodiment of the present disclosure. The system may be used system for secure handling of sensitive data. The system comprises: a sensing device configured to measure electrophysiological signals and to encrypt the measured electrophysiological signals to generate encrypted electrophysiological signals using a public data key; a remote server configured to receive the encrypted electrophysiological signals from the sensing device, wherein the remote server is further configured to decrypt the encrypted electrophysiological signals based on a user's key phrase and a private data key to generate classified data based on the decrypted electrophysiological signals
In an embodiment, the system further comprises: a user device configured to receive the encrypted electrophysiological signals from the sensing device and transmit the encrypted electrophysiological signals to the remote server.

In an embodiment, the remote server is further configured to display the classified data on a user interface.

In an embodiment, the remote server is further configured to transmit the classified data to a user device.

In an embodiment, the user device comprises a display and wherein the user device is configured to display the classified data on the display.

In an embodiment, the remote server is further configured to receive instructions on how to generate the classified data, wherein the instructions preferably comprise information on one or more classifications from a set of classifications that are to be generated as the classified data.

In an embodiment, the remote server is further configured to generate a token based on the user's key phrase and/or the classified data, wherein the token is preferably indicative of a successful reception of the user's key phrase and/or indicative on how to generate the classified data.

In an embodiment, the token comprises a predetermined or user specified validation time, wherein upon expiration of the validation time the remote server is configured to stop generating classified data.

In an embodiment, before the sensing device is used to measure electrophysiological signals, the sensing device comprises an initial public data key and the remote server comprises an initial private data key, wherein the initial private and public data keys are encrypted by a default random-generated device password, and wherein the remote server is configured to: generate a new encrypted private data key and a new public data key upon change, by a user, of the default random-generated device password to the user's key phrase; send the new public data key to the sensing device as the public data key; and store the new private data key on the external server as the private data key.

In an embodiment, the user's key phrase is specific for a particular sensing device.

Fig. 3 shows a schematic diagram of a method according to an embodiment of the present disclosure. The method may be used for secure handling of sensitive data. The method includes the following steps:
S301: measuring electrophysiological signals using a sensing device;
S302: encrypting the measured electrophysiological signals by means of a public data key provided on the sensing device;
S303: transmitting, by the sensing device, the encrypted electrophysiological signals to a remote server;
S304: receiving, by the remote server, the encrypted electrophysiological signals;
S305: decrypting the encrypted electrophysiological signals at the remote server based on a user's key phrase and a private data key; and
S306: generating classified data based on the decrypted electrophysiological signals at the remote server.

In an embodiment, The method of claim 11, wherein transmitting, by the sensing device, the encrypted electrophysiological signals to a remote server comprises transmitting the encrypted electrophysiological signals via a user device.

In an embodiment, the method further comprises: displaying, by the remote server, the classified data on a user interface.

In an embodiment, the method further comprises: transmitting, by the remote server, the classified data to a user device.

In an embodiment, further comprises: displaying, by the user device, the classified data.

In an embodiment, generating classified data based on the encrypted electrophysiological signals at the remote server comprises: receiving, by the remote server, instructions on how to generate the classified data, wherein the instructions preferably comprise information on one or more classifications from a set of classifications that are to be generated as the classified data.

In an embodiment, the method further comprises: generating a token based on the user's key phrase and/or the classified data, wherein the token is preferably indicative of a successful reception of the user's key phrase and/or indicative on how to generate the classified data.

In an embodiment, the token comprises a predetermined or user specified validation time, wherein the method further comprises: stopping, by the remote server, to generating classified data upon expiration of the validation time.

In an embodiment, before the sensing device is used to measure electrophysiological signals, the method comprises: generating an initial private data key for the remote server and an initial public data key for the sensing device; encrypting the initial private and public data keys by a default random-generated device password; generating a new encrypted private data key and a new public data key upon change, by a user, of the default random-generated device password to the user's key phrase; storing the new public data key on the sensing device as the public data key; and storing the new private data key on the external server as the private data key.

In an embodiment, the user's key phrase is specific for a particular sensing device.

In an embodiment (not shown), a computer-implemented method comprising the steps of the above described method for secure handling of sensitive data is provided.

In an embodiment (not shown), a computer program product comprising instructions which, when the program is executed by a computer, cause the computer to carry out the steps of the above described method for secure handling of sensitive data is provided.

In view of the systems and methods described herein, third-parties may use the sensing device to provide extended applications without users having to download drivers or additional background applications. The raw data flows directly through their apps without providing access to the raw data, protecting users' privacy. Users can gradually opt into different classifications, i.e. individually choose different classifications, which are then securely and encrypted at the external server 20, ensuring that the third-parties can only access users' classified data if they have permission to do so. In addition, the data is only processed in certain ways if the user gives permission and the provider of the sensing device 10 and/or the externals server 20 has no control over the streamed and stored data classification if the user does not allow it. That is, users have a public key on their hardware (sensing device 10) to encrypt the raw data via hardware acceleration and an encrypted private key is provided on the external server 20 that can only be decrypted with a user key phrase to decrypt the raw data.

In other words, the opt-in of the various classifications cannot be bypassed by the third-party since this is handled by the user on a provided web application from the external server 20. The third-party has no access to the raw data since they have no access to the private data key and the user key phrase. Even if the third-party would get access to the key phrase, the third-party can still not decrypt the brain data, because of the missing private data key. In addition, the provider of the sensing device 10 and/or the external server 20 has no access to the raw data if the user has not given an opt-in and key phrase in order to decrypt the data on the external server 20. Users have no access to the raw data since they need to have access to the private data key which is only available on the external server 20 and never gets sent. If users do not register for an API key, they cannot even send data to the external server 20. There is no need to download an additional application or driver to make this work, all of this can be implemented by including a lightweight SDK into the third-party application.

Thus, unobtrusive systems and methods are provided that can be integrated into a range of applications to extend their functionality with health related data sensing with enabling the least privilege over the data: no raw data access to third-parties, no control of raw data from the provider of the sensing device 10 and/or the external server 20 and full ownership of the raw data from the user based on opt-in procedure. The least privilege in this context means: users have full control over their data, third-parties can access classified data from the raw data, but not the raw data itself, and the provider of the sensing device 10 and/or the external server 20 has access to the raw data but no control over it if there's no key phrase and opt-in from the user.

While various embodiments of the present disclosure have been described above, it should be understood that they have been presented by way of example only, and not by way of limitation. Likewise, the various diagrams may depict an example architectural or configuration, which are provided to enable persons of ordinary skill in the art to understand exemplary features and functions of the present disclosure. Such persons would understand, however, that the present disclosure is not restricted to the illustrated example architectures or configurations, but can be implemented using a variety of alternative architectures and configurations. Additionally, as would be understood by persons of ordinary skill in the art, one or more features of one embodiment can be combined with one or more features of another embodiment described herein. Thus, the breadth and scope of the present disclosure should not be limited by any one of the above-described exemplary embodiments.

It is also understood that any reference to an element herein using a designation such as "first," "second," and so forth does not generally limit the quantity or order of those elements. Rather, these designations can be used herein as a convenient means of distinguishing between two or more elements or instances of an element. Thus, a reference to first and second elements does not mean that only two elements can be employed, or that the first element must precede the second element in some manner.

Additionally, a person having ordinary skill in the art would understand that information and signals can be represented using any one of a variety of different technologies and techniques. For example, data, instructions, commands, information, signals, bits and symbols, for example, which may be referenced in the above description can be represented by voltages, currents, electromagnetic waves, magnetic fields or particles, optical fields or particles, or any combination thereof.

A skilled person would further appreciate that any one of the various illustrative logical blocks, units, processors, means, circuits, methods and functions described in connection with the aspects disclosed herein can be implemented by electronic hardware (e.g., a digital implementation, an analog implementation, or a combination of the two), firmware, various forms of program or design code incorporating instructions (which can be referred to herein, for convenience, as "software" or a "software unit"), or any combination of these techniques.

To clearly illustrate this interchangeability of hardware, firmware and software, various illustrative components, blocks, units, circuits, and steps have been described above generally in terms of their functionality. Whether such functionality is implemented as hardware, firmware or software, or a combination of these techniques, depends upon the particular application and design constraints imposed on the overall system. Skilled artisans can implement the described functionality in various ways for each particular application, but such implementation decisions do not cause a departure from the scope of the present disclosure. In accordance with various embodiments, a processor, device, component, circuit, structure, machine, unit, etc. can be configured to perform one or more of the functions described herein. The term "configured to" or "configured for" as used herein with respect to a specified operation or function refers to a processor, device, component, circuit, structure, machine, unit, etc. that is physically constructed, programmed and/or arranged to perform the specified operation or function.

Furthermore, a skilled person would understand that various illustrative logical blocks, units, devices, components and circuits described herein can be implemented within or performed by an integrated circuit (IC) that can include a general purpose processor, a digital signal processor (DSP), an application specific integrated circuit (ASIC), a field programmable gate array (FPGA) or other programmable logic device, or any combination thereof. The logical blocks, units, and circuits can further include antennas and/or transceivers to communicate with various components within the device. A general purpose processor can be a microprocessor, but in the alternative, the processor can be any conventional processor, controller, or state machine. A processor can also be implemented as a combination of computing devices, e.g., a combination of a DSP and a microprocessor, a plurality of microprocessors, one or more microprocessors in conjunction with a DSP core, or any other suitable configuration to perform the functions described herein. If implemented in software, the functions can be stored as one or more instructions or code on a computer-readable medium. Thus, the steps of a method or algorithm disclosed herein can be implemented as software stored on a computer-readable medium.

Computer-readable media includes both computer storage media and communication media including any medium that can be enabled to transfer a computer program or code from one place to another. A storage media can be any available media that can be accessed by a computer. By way of example, and not limitation, such computer-readable media can include RAM, ROM, EEPROM, CD-ROM or other optical disk storage, magnetic disk storage or other magnetic storage devices, or any other medium that can be used to store desired program code in the form of instructions or data structures and that can be accessed by a computer.

In this document, the term "unit" as used herein, refers to software, firmware, hardware, and any combination of these elements for performing the associated functions described herein. Additionally, for purpose of discussion, the various units are described as discrete units; however, as would be apparent to one of ordinary skill in the art, two or more units may be combined to form a single unit that performs the associated functions according to embodiments of the present disclosure.

Additionally, memory or other storage, as well as communication components, may be employed in embodiments of the present disclosure. It will be appreciated that, for clarity purposes, the above description has described embodiments of the present disclosure with reference to different functional units and processors. However, it will be apparent that any suitable distribution of functionality between different functional units, processing logic elements or domains may be used without detracting from the present disclosure. For example, functionality illustrated to be performed by separate processing logic elements, or controllers, may be performed by the same processing logic element, or controller. Hence, references to specific functional units are only references to a suitable means for providing the described functionality, rather than indicative of a strict logical or physical structure or organization.

Various modifications to the implementations described in this disclosure will be readily apparent to those skilled in the art, and the general principles defined herein can be applied to other implementations without departing from the scope of the claims. Thus, the disclosure is not intended to be limited to the implementations shown herein, but is to be accorded the widest scope consistent with the novel features and principles disclosed herein, as recited in the claims below.

## Claims

1. A system for secure handling of sensitive data, the system comprising:
a sensing device (10) configured to measure electrophysiological signals and to encrypt the measured electrophysiological signals to generate encrypted electrophysiological signals using a public data key (11);
a remote server (20) configured to receive the encrypted electrophysiological signals from the sensing device (10), and to store an encrypted private data key (21),
wherein the remote server (20) is further configured to decrypt the private data key (21) based on a user's key phrase and to decrypt the encrypted electrophysiological signals based on the decrypted private data key (21) to generate classified data based on the decrypted electrophysiological signals.

2. The system of claim 1, further comprising:
a user device (30) configured to receive the encrypted electrophysiological signals from the sensing device (10) and transmit the encrypted electrophysiological signals to the remote server (20).

3. The system of claim 1 or 2, wherein the remote server (20) is further configured to display the classified data on a user interface; and/or wherein the remote server (20) is further configured to transmit the classified data to a user device (30), and preferably the user device (30) comprises a display and wherein the user device (30) is configured to display the classified data on the display.

4. The system of any one of claims 1 to 3, wherein the remote server (20) is further configured to receive instructions on how to generate the classified data, wherein the instructions preferably comprise information on one or more classifications from a set of classifications that are to be generated as the classified data.

5. The system of any one of claims 1 to 4, wherein the remote server (20) is further configured to generate a token based on the user's key phrase and/or the classified data, wherein the token is preferably indicative of a successful reception of the user's key phrase and/or indicative on how to generate the classified data, and preferably wherein the token comprises a predetermined or user specified validation time, wherein upon expiration of the validation time the remote server (20) is configured to stop generating classified data.

6. The system of any one of claims 1 to 5, wherein, before the sensing device (10) is used to measure electrophysiological signals, the sensing device (10) comprises an encrypted initial public data key and the remote server (20) comprises an encrypted initial private data key, wherein the remote server (20) is configured to:
generate a new encrypted private data key and a new public data key upon change, by a user, of a default random-generated device password to the user's key phrase;
send the new public data key to the sensing device (10) as the public data key (11); and
store the new private data key on the external server (20) as the private data key (21).

7. The system of any one of claims 1 to 6, wherein the user's key phrase is specific for a particular sensing device (10).

8. A method for secure handling of sensitive data, the method including the following steps:
measuring electrophysiological signals using a sensing device (10);
encrypting the measured electrophysiological signals by means of a public data key (11) provided on the sensing device (10);
transmitting, by the sensing device (10), the encrypted electrophysiological signals to a remote server (20);
receiving, by the remote server (20), the encrypted electrophysiological signals;
decrypting an encrypted private data key (21) provided on the remote server (20) based on a user's key phrase;
decrypting the encrypted electrophysiological signals at the remote server (20) based on the decrypted private data key (21);
generating classified data based on the decrypted electrophysiological signals at the remote server (20).

9. The method of claim 8, wherein transmitting, by the sensing device (10), the encrypted electrophysiological signals to a remote server (20) comprises transmitting the encrypted electrophysiological signals via a user device (30).

10. The method of claim 8 or 9, further comprising:
displaying, by the remote server (20), the classified data on a user interface; and/or
transmitting, by the remote server (20), the classified data to a user device (30), and preferably comprising:
displaying, by the user device (10), the classified data.

11. The method of any one of claims 8 to 10, wherein generating classified data based on the encrypted electrophysiological signals at the remote server (20) comprises:
receiving, by the remote server (20), instructions on how to generate the classified data, wherein the instructions preferably comprise information on one or more classifications from a set of classifications that are to be generated as the classified data.

12. The method of any one of claims 8 to 11, further comprising:
generating, by the remote server (20), a token based on the user's key phrase and/or the classified data, wherein the token is preferably indicative of a successful reception of the user's key phrase and/or indicative on how to generate the classified data, and preferably
wherein the token comprises a predetermined or user specified validation time, wherein the method further comprises:
stopping, by the remote server (20), to generating classified data upon expiration of the validation time.

13. The method of any one of claims 8 to 12, before the sensing device (10) is used to measure electrophysiological signals, the method comprises:
generating an initial private data key for the remote server (20) and an initial public data key for the sensing device (10);
encrypting the initial private and public data keys by a default random-generated device password;
generating, by the remote server (20), a new encrypted private data key and a new public data key upon change, by a user, of the default random-generated device password to the user's key phrase;
storing, by the sensing device (10), the new public data key on the sensing device (10) as the public data key (11); and
storing, by the external server (20), the new private data key on the external server (20) as the private data key (21).

14. The method of any one of claims 8 to 13, wherein the user's key phrase is specific for a particular sensing device.

## Patentansprüche

1. System zum sicheren Umgang mit sensiblen Daten, wobei das System umfasst:
ein Erfassungsgerät (10), das dazu ausgelegt ist, elektrophysiologische Signale zu messen und die gemessenen elektrophysiologischen Signale unter Verwendung eines öffentlichen Datenschlüssels (11) zu verschlüsseln, um verschlüsselte elektrophysiologische Signale zu erzeugen;
einen Fernserver (20), der dazu ausgelegt ist, die verschlüsselten elektrophysiologischen Signale vom Erfassungsgerät (10) zu empfangen und einen verschlüsselten privaten Datenschlüssel (21) zu speichern,
wobei der Fernserver (20) ferner dazu ausgelegt ist, den privaten Datenschlüssel (21) basierend auf dem Schlüsselsatz eines Nutzers zu entschlüsseln und die verschlüsselten elektrophysiologischen Signale basierend auf dem entschlüsselten privaten Datenschlüssel (21) zu entschlüsseln, um klassifizierte Daten basierend auf den entschlüsselten elektrophysiologischen Signalen zu erzeugen.

2. System nach Anspruch 1, ferner umfassend:
ein Nutzergerät (30), das dazu ausgelegt ist, die verschlüsselten elektrophysiologischen Signale vom Erfassungsgerät (10) zu empfangen und die verschlüsselten elektrophysiologischen Signale an den Fernserver (20) zu übermitteln.

3. System nach Anspruch 1 oder 2, wobei der Fernserver (20) ferner dazu ausgelegt ist, die klassifizierten Daten auf einer Nutzeroberfläche anzuzeigen; und/oder wobei der Fernserver (20) ferner dazu ausgelegt ist, die klassifizierten Daten an ein Nutzergerät (30) zu übermitteln, und vorzugsweise das Nutzergerät (30) eine Anzeige umfasst und wobei das Nutzergerät (30) dazu ausgelegt ist, die klassifizierten Daten auf der Anzeige anzuzeigen.

4. System nach einem der Ansprüche 1 bis 3, wobei der Fernserver (20) ferner dazu ausgelegt ist, Anweisungen darüber zu empfangen, wie die klassifizierten Daten zu erzeugen sind, wobei die Anweisungen vorzugsweise Informationen über eine oder mehrere Klassifikationen aus einem Satz von Klassifikationen enthalten, die als die klassifizierten Daten zu erzeugen sind.

5. System nach einem der Ansprüche 1 bis 4, wobei der Fernserver (20) ferner dazu ausgelegt ist, ein Token basierend auf dem Schlüsselsatz des Nutzers und/oder den klassifizierten Daten zu erzeugen, wobei das Token vorzugsweise einen erfolgreichen Empfang des Schlüsselsatzes des Nutzers anzeigt und/oder anzeigt, wie die klassifizierten Daten zu erzeugen sind, und vorzugsweise wobei das Token eine vorbestimmte oder nutzerspezifizierte Validierungszeit umfasst, wobei nach Ablauf der Validierungszeit der Fernserver (20) dazu ausgelegt ist, das Erzeugen klassifizierter Daten zu stoppen.

6. System nach einem der Ansprüche 1 bis 5, wobei vor dem Verwenden des Erfassungsgeräts (10) zum Messen elektrophysiologischer Signale, das Erfassungsgerät (10) einen verschlüsselten anfänglich öffentlichen Datenschlüssel enthält und der Fernserver (20) einen verschlüsselten anfänglich privaten Datenschlüssel enthält, wobei der Fernserver (20) dazu ausgelegt ist,
einen neuen verschlüsselten privaten Datenschlüssel und einen neuen öffentlichen Datenschlüssel zu erzeugen, und zwar nach Abänderung eines voreingestellten zufallsgenerierten Gerätepassworts durch einen Nutzer auf den Schlüsselsatz des Nutzers;
den neuen öffentlichen Datenschlüssel als den öffentlichen Datenschlüssel (11) an das Erfassungsgerät (10) zu senden; und
den neuen privaten Datenschlüssel als den privaten Datenschlüssel (21) auf dem externen Server (20) zu speichern.

7. System nach einem der Ansprüche 1 bis 6, wobei der Schlüsselsatz des Nutzers für ein bestimmtes Erfassungsgerät (10) spezifisch ist.

8. Verfahren zum sicheren Umgang mit sensiblen Daten, wobei das Verfahren die folgenden Schritte umfasst:
Messen elektrophysiologischer Signale mittels eines Erfassungsgeräts (10);
Verschlüsseln der gemessenen elektrophysiologischen Signale mittels eines auf dem Erfassungsgerät (10) bereitgestellten öffentlichen Datenschlüssels (11);
Übermitteln, durch das Erfassungsgerät (10), der verschlüsselten elektrophysiologischen Signale an einen Fernserver (20);
Empfangen, durch den Fernserver (20), der verschlüsselten elektrophysiologischen Signale;
Entschlüsseln eines auf dem Fernserver (20) bereitgestellten verschlüsselten privaten Datenschlüssels (21) basierend auf dem Schlüsselsatz eines Nutzers;
Entschlüsseln der verschlüsselten elektrophysiologischen Signale am Fernserver (20) basierend auf dem entschlüsselten privaten Datenschlüssel (21);
Erzeugen klassifizierter Daten basierend auf den entschlüsselten elektrophysiologischen Signalen am Fernserver (20).

9. Verfahren nach Anspruch 8, wobei das Übermitteln, durch das Erfassungsgerät (10), der verschlüsselten elektrophysiologischen Signale an einen Fernserver (20) das Übermitteln der verschlüsselten elektrophysiologischen Signale über ein Nutzergerät (30) umfasst.

10. Verfahren nach Anspruch 8 oder 9, ferner umfassend:
Anzeigen, durch den Fernserver (20), der klassifizierten Daten auf einer Nutzerschnittstelle; und/oder
Übermitteln, durch den Fernserver (20), der klassifizierten Daten an ein Nutzergerät (30), und vorzugsweise umfassend:
Anzeigen, durch das Nutzergerät (30), der klassifizierten Daten.

11. Verfahren nach einem der Ansprüche 8 bis 10, wobei das Erzeugen klassifizierter Daten basierend auf den entschlüsselten elektrophysiologischen Signalen am Fernserver (20) Folgendes umfasst:
Empfangen, durch den Fernserver (20), von Anweisungen darüber, wie die klassifizierten Daten zu erzeugen sind, wobei die Anweisungen vorzugsweise Informationen über eine oder mehrere Klassifikationen aus einem Satz von Klassifikationen enthalten, die als die klassifizierten Daten zu erzeugen sind.

12. Verfahren nach einem der Ansprüche 8 bis 11, ferner umfassend:
Erzeugen, durch den Fernserver (20), eines Tokens basierend auf dem Schlüsselsatz des Nutzers und/oder den klassifizierten Daten, wobei das Token vorzugsweise einen erfolgreichen Empfang des Schlüsselsatzes des Nutzers anzeigt und/oder anzeigt, wie die klassifizierten Daten zu erzeugen sind, und vorzugsweise
wobei das Token eine vorbestimmte oder nutzerspezifizierte Validierungszeit umfasst, wobei das Verfahren ferner umfasst:
Stoppen, durch den Fernserver (20), des Erzeugens klassifizierter Daten nach Ablauf der Validierungszeit.

13. Verfahren nach einem der Ansprüche 8 bis 12, wobei vor dem Verwenden des Erfassungsgeräts (10) zum Messen elektrophysiologischer Signale, das Verfahren umfasst:
Erzeugen eines anfänglich privaten Datenschlüssels für den Fernserver (20) und eines anfänglich öffentlichen Datenschlüssels für das Erfassungsgerät (10);
Verschlüsseln der anfänglich privaten und öffentlichen Datenschlüssel mit einem voreingestellten zufallsgenerierten Gerätepasswort;
Erzeugen, durch den Fernserver (20), eines neuen verschlüsselten privaten Datenschlüssels und eines neuen öffentlichen Datenschlüssels nach Abänderung des voreingestellten zufallsgenerierten Gerätepassworts durch einen Nutzer auf den Schlüsselsatz des Nutzers;
Speichern, durch das Erfassungsgerät (10), des neuen öffentlichen Datenschlüssels als den öffentlichen Datenschlüssel (11) auf dem Erfassungsgerät (10); und
Speichern, durch den externen Server (20), des neuen privaten Datenschlüssels als den privaten Datenschlüssel (21) auf dem externen Server (20).

14. Verfahren nach einem der Ansprüche 8 bis 13, wobei der Schlüsselsatz des Nutzers für ein bestimmtes Erfassungsgerät spezifisch ist.

## Revendications

1. Système de gestion sécurisée de données sensibles, le système comprenant :
un dispositif de détection (10) configuré pour mesurer des signaux électrophysiologiques et pour chiffrer les signaux électrophysiologiques mesurés pour générer des signaux électrophysiologiques chiffrés en utilisant une clé de données publique (11) ;
un serveur distant (20) configuré pour recevoir les signaux électrophysiologiques chiffrés en provenance du dispositif de détection (10) et pour stocker une clé de données privée chiffrée (21),
dans lequel le serveur distant (20) est en outre configuré pour déchiffrer la clé de données privée (21) sur la base d'une phrase-clé d'utilisateur et pour déchiffrer les signaux électrophysiologiques chiffrés sur la base de la clé de données privée (21) pour générer des données classées sur la base des signaux électrophysiologiques déchiffrés.

2. Système selon la revendication 1, comprenant en outre :
un dispositif utilisateur (30) configuré pour recevoir les signaux électrophysiologiques chiffrés depuis le dispositif de détection (10) et transmettre les signaux électrophysiologiques chiffrés au serveur distant (20).

3. Système selon la revendication 1 ou 2, dans lequel le serveur distant (20) est en outre configuré pour afficher les données classées sur une interface utilisateur ; et/ou dans lequel le serveur distant (20) est en outre configuré pour transmettre les données classées à un dispositif utilisateur (30), et de préférence le dispositif utilisateur (30) comprend un affichage et dans lequel le dispositif utilisateur (30) est configuré pour afficher les données classées sur l'affichage.

4. Système selon l'une quelconque des revendications 1 à 3, dans lequel le serveur distant (20) est en outre configuré pour recevoir des instructions sur la manière de générer les données classées, dans lequel les instructions comprennent de préférence des informations sur une ou plusieurs classifications à partir d'un ensemble de classifications qui doivent être générées en tant que données classées.

5. Système selon l'une quelconque des revendications 1 à 4, dans lequel le serveur distant (20) est en outre configuré pour générer un jeton sur la base de la phrase-clé d'utilisateur et/ou des données classées, dans lequel le jeton indique de préférence une réception réalisée avec succès de la phrase-clé d'utilisateur et/ou indique la manière de générer les données classées, et de préférence dans lequel le jeton comprend un temps de validation prédéterminé ou spécifié par l'utilisateur, dans lequel à l'expiration du temps de validation, le serveur distant (20) est configuré pour arrêter de générer des données classées.

6. Système selon l'une quelconque des revendications 1 à 5, dans lequel, avant que le dispositif de détection (10) ne soit utilisé pour mesurer des signaux électrophysiologiques, le dispositif de détection (10) comprend une clé de données publique initiale et le serveur distant (20) comprend une clé de données privée initiale chiffrée, dans lequel le serveur distant (20) est configuré pour :
générer une nouvelle clé de données privée chiffrée et une nouvelle clé de données publique lors du changement, par un utilisateur, d'un mot de passe de dispositif généré de manière aléatoire par défaut en la phrase-clé d'utilisateur ;
envoyer la nouvelle clé de données publique au dispositif de détection (10) en tant que clé de données publique (11) ; et
stocker la nouvelle clé de données privée sur le serveur externe (20) en tant que clé de données privée (21).

7. Système selon l'une quelconque des revendications 1 à 6, dans lequel la phrase-clé d'utilisateur est spécifique à un dispositif de détection particulier (10).

8. Procédé de gestion sécurisée de données sensibles, le procédé comportant les étapes suivantes :
de mesure de signaux électrophysiologiques en utilisant un dispositif de détection (10) ;
de chiffrement des signaux électrophysiologiques mesurés au moyen d'une clé publique de données (11) prévue sur le dispositif de détection (10) ;
de transmission, par le dispositif de détection (10), des signaux électrophysiologiques chiffrés à un serveur distant (20) ;
de réception, par le serveur distant (20), des signaux électrophysiologiques chiffrés ;
de déchiffrement d'une clé de données privée chiffrée (21) prévue sur le serveur distant (20) sur la base d'une phrase-clé d'utilisateur ;
de déchiffrement des signaux électrophysiologiques chiffrés sur le serveur distant (20) sur la base de la clé de données privée déchiffrée (21) ;
de génération de données classées sur la base des signaux électrophysiologiques déchiffrés sur le serveur distant (20).

9. Procédé selon la revendication 8, dans lequel la transmission, par le dispositif de détection (10), des signaux électrophysiologiques chiffrés à un serveur distant (20) comprend la transmission des signaux électrophysiologiques chiffrés par l'intermédiaire d'un dispositif utilisateur (30).

10. Procédé selon la revendication 8 ou 9, comprenant en outre :
l'affichage, par le serveur distant (20), des données classées sur une interface utilisateur ; et/ou
la transmission, par le serveur distant (20), des données classées à un dispositif utilisateur (30), et comprenant de préférence :
l'affichage, par le dispositif utilisateur (30), des données classées.

11. Procédé selon l'une quelconque des revendications 8 à 10, dans lequel la génération de données classées sur la base des signaux électrophysiologiques chiffrés sur le serveur distant (20) comprend :
la réception, par le serveur distant (20), d'instructions sur la manière de générer les données classées, dans lequel les instructions comprennent de préférence des informations sur une ou plusieurs classifications provenant d'un ensemble de classifications qui doivent être générées comme les données classées.

12. Procédé selon l'une quelconque des revendications 8 à 11, comprenant en outre :
la génération, par le serveur distant (20), d'un jeton sur la base de la phrase-clé d'utilisateur et/ou des données classées, dans lequel le jeton indique de préférence une réception réalisée avec succès de la phrase-clé d'utilisateur et/ou indique la manière de générer les données classées, et de préférence
dans lequel le jeton comprend un temps de validation prédéterminé ou spécifié par l'utilisateur, dans lequel le procédé comprend en outre :
l'arrêt, par le serveur distant (20), de la génération de données classées à l'expiration du temps de validation.

13. Procédé selon l'une quelconque des revendications 8 à 12, avant que le dispositif de détection (10) ne soit utilisé pour mesurer des signaux électrophysiologiques, le procédé comprenant :
la génération d'une clé de données privée initiale pour le serveur distant (20) et d'une clé de données publique initiale pour le dispositif de détection (10) ;
le chiffrement des clés de données privées et publiques initiales par un mot de passe de dispositif généré de manière aléatoire par défaut ;
la génération, par le serveur distant (20), d'une nouvelle clé de données privée chiffrée et d'une nouvelle clé de données publique lors du changement, par un utilisateur, du mot de passe de dispositif généré de manière aléatoire par défaut en la phrase-clé d'utilisateur ;
le stockage, par le dispositif de détection (10), de la nouvelle clé publique de données sur le dispositif de détection (10) en tant que la clé de données publique (11) ; et
le stockage, par le serveur externe (20), de la nouvelle clé de données privée sur le serveur externe (20) en tant que la clé de données privée (21).

14. Procédé selon l'une quelconque des revendications 8 à 13, dans lequel la phrase-clé d'utilisateur est spécifique à un dispositif de détection particulier.
